(19) **Europäisches Patentamt / European Patent Office / Office européen des brevets**

(11) **EP 4 149 488 B1**

(12) # EUROPEAN PATENT SPECIFICATION

(45) Date of publication and mention of the grant of the patent:
**15.04.2026 Bulletin 2026/16**

(21) Application number: **21723774.2**

(22) Date of filing: **11.05.2021**

(51) International Patent Classification (IPC):
*A61K 38/38* (2006.01)   *A61K 31/727* (2006.01)
*A61K 47/64* (2017.01)   *A61K 47/50* (2017.01)
*A61P 7/04* (2006.01)   *A61P 7/02* (2006.01)

(52) Cooperative Patent Classification (CPC):
**A61K 31/727; A61K 38/385; A61K 47/643; A61P 7/02; A61P 7/04**

(86) International application number:
**PCT/EP2021/062501**

(87) International publication number:
**WO 2021/228866 (18.11.2021 Gazette 2021/46)**

(54) **AN ANTITHROMBIC MOLECULE HAVING APAC ACTIVITY FOR USE IN THE PREVENTION AND/ OR TREATMENT OF THROMBOCYTOPENIA**

ANTITHROMBISCHES MOLEKÜL MIT APAC-AKTIVITÄT ZUR VERWENDUNG IN DER PRÄVENTION UND/ODER BEHANDLUNG VON THROMBOZYTOPENIE

MOLÉCULE ANTITHROMBIQUE AYANT UNE ACTIVITÉ APAC POUR SON UTILISATION DANS LA PRÉVENTION ET/OU LE TRAITEMENT DE LA THROMBOCYTOPÉNIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2020 GB 202006960**

(43) Date of publication of application:
**22.03.2023 Bulletin 2023/12**

(73) Proprietor: **Aplagon OY**
**00290 Helsinki (FI)**

(72) Inventor: **LASSILA, Riitta**
**00290 Helsinki (FI)**

(74) Representative: **Symbiosis IP Limited**
**Unit 5C**
**Sbarc I Spark Building**
**Maindy Road**
**Cardiff CF24 4HQ (GB)**

(56) References cited:
**WO-A1-2016/030316   WO-A2-2006/047755**

- **N. V. RAO ET AL: "Low anticoagulant heparin targets multiple sites of inflammation, suppresses heparin-induced thrombocytopenia, and inhibits interaction of RAGE with its ligands", AMERICAN JOURNAL OF PHYSIOLOGY CELL PHYSIOLOGY, vol. 299, no. 1, 7 April 2010 (2010-04-07), US, pages C97 - C110, XP055339841, ISSN: 0363-6143, DOI: 10.1152/ajpcell.00009.2010**
- **RIITTA LASSILA ET AL: "Mast Cell-Derived Heparin Proteoglycans As a Model for a Local Antithrombotic", SEMINARS IN THROMBOSIS AND HEMOSTASIS, vol. 40, no. 08, 27 November 2014 (2014-11-27), US, pages 837 - 844, XP055767070, ISSN: 0094-6176, DOI: 10.1055/ s-0034-1395157**
- **TUUMINEN RAIMO ET AL: "Dual antiplatelet and anticoagulant APAC prevents experimental ischemia-reperfusion-induced acute kidney injury", CLINICAL AND EXPERIMENTAL NEPHROLOGY, JAPANESE SOCIETY OF NEPHROLOGY, TOKYO, JP, vol. 21, no. 3, 12 July 2016 (2016-07-12), pages 436 - 445, XP036254678, ISSN: 1342-1751, [retrieved on 20160712], DOI: 10.1007/S10157-016-1308-2**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 149 488 B1

**(Cont. next page)**

- JASON CHEN ET AL: "Dual antiplatelet and anticoagulant (APAC) heparin proteoglycan mimetic with shear-dependent effects on platelet-collagen binding and thrombin generation", THROMBOSIS RESEARCH, vol. 169, 1 September 2018 (2018-09-01), AMSTERDAM, NL, pages 143 - 151, XP055766470, ISSN: 0049-3848, DOI: 10.1016/j.thromres.2018.07.026
- AREPALLY G ET AL: "HEPARIN-INDUCED THROMBOCYTOPENIA AND THROMBOSIS", CLINICAL REVIEWS IN ALLERGY AND IMMUNOLOGY, HUMANA PRESS, TOTOWA, NJ, US, vol. 16, no. 3, 1 January 1998 (1998-01-01), pages 237 - 247, XP000942861, ISSN: 1080-0549
- IVASCU NATALIA S ET AL: "Heparin-Induced Thrombocytopenia: A Review for Cardiac Anesthesiologists and Intensivists", JOURNAL OF CARDIOTHORACIC AND VASCULAR ANESTHESIA, ELSEVIER, AMSTERDAM, NL, vol. 33, no. 2, 26 October 2018 (2018-10-26), pages 511 - 520, XP085575819, ISSN: 1053-0770, DOI: 10.1053/J.JVCA.2018.10.035
- HUANG HE ET AL: "Successful Treatment of Severe Heparin-induced Thrombocytopenia with Intravenous Immunoglobulin, Platelet Transfusion and Rivaroxaban: A Case Report", CHINESE MEDICAL SCIENCES JOURNAL, BEIJING, CN, vol. 34, no. 1, 1 March 2019 (2019-03-01), pages 60 - 64, XP085912378, ISSN: 1001-9294, [retrieved on 20191118], DOI: 10.24920/003462
- SHARIFI MOHSEN ET AL: "New Oral Anticoagulants in the Treatment of Heparin-Induced Thrombocytopenia", THROMBOSIS RESEARCH, vol. 135, no. 4, 10 January 2015 (2015-01-10), pages 607 - 609, XP029203409, ISSN: 0049-3848, DOI: 10.1016/J.THROMRES.2015.01.009
- THALER JOHANNES ET AL: "Successful treatment of vaccine-induced prothrombotic immune thrombocytopenia (VIPIT)", JOURNAL OF THROMBOSIS AND HAEMOSTASIS, vol. 19, no. 7, 1 July 2021 (2021-07-01), GB, pages 1819 - 1822, XP055822399, ISSN: 1538-7933, Retrieved from the Internet <URL:https://www.aniara.com/mm5/PDFs/Literature/HBM_jth.15346.pdf> DOI: 10.1111/jth.15346

**Description**

**Field of the Invention**

The invention relates to an anti-thrombotic molecule having both anti-platelet and anticoagulant (APAC) activity for use in the prevention and/or treatment heparin-induced thrombocytopenia (HIT) type I or II; and/or heparin-induced thrombocytopenia and thrombosis (HITT); and/or heparin-independent thrombocytopenia autoimmune HIT (aHIT); and/or vaccine-induced thrombocytopenia and thrombosis (VITT). The invention has use in both the medical and veterinary industries.

**Background of the Invention**

Thrombocytopenia can result from conditions that lead to increased platelet destruction or decreased platelet production. Heparin can cause thrombocytopenia via immune and non-immune mediated mechanisms. These two types of heparin-induced thrombocytopenia (HIT) can result from heparin administration: type I, non-immune-mediated; and type II, immune-mediated. The term 'non-immune heparin-associated thrombocytopenia' is used to denote type I, a benign condition in which no heparin-dependent antibodies are present. The term 'immune-mediated heparin-induced thrombocytopenia' (HIT) is used to denote thrombocytopenia in which pathogenic heparin-dependent antibodies are detectable, this term is the most widely accepted designation for HIT type II.

It also has become recognized that some patients present with clinical symptoms and laboratory features of HIT despite not having previously received heparin, either in the recent past or at all (spontaneous HIT syndrome). Sera from these patients contain antibodies that activate platelets strongly even in the absence of heparin. However, such 'heparin-independent' platelet-activating properties are not unique to spontaneous HIT syndrome but are also found in sera of a minority of (heparin-dependent) typical HIT patients. Moreover, patients who show this *in vitro* reactivity profile are more likely to have unusual HIT syndromes such as delayed-onset HIT, persisting HIT, fondaparinux- associated HIT, and HIT induced by exposure to heparin 'flushes'. This form of HIT is termed aHIT. More recently, aHIT-like, PF4-dominating antibodies have been recognized also in vaccine-induced thrombocytopenia and thrombosis (VITT).

Additionally, any type of thrombocytopenia (HIT I, HIT II, aHIT or VITT) may give rise to a thrombosis and so a patient may present with thrombocytopenia and thrombosis (HITT). HITT, notably, can cause either an arterial or a venous thrombosis and occur at multiple sites.

In summary, HIT I is heparin-associated and transient, whereas HIT II is immunological, longstanding and more morbid, as further HIT II antibodies can predispose to HITT.

Heparin-induced thrombocytopenia (HIT type II) is a dangerous, potentially lethal, immunological response to unfractionated heparin (UFH) or, less commonly, low molecular weight heparin (LMWH).

The prevalence of HIT II ranges from 0.1-5% of patients receiving heparin with 35-50% of those patients developing thrombosis and so exhibiting HITT. The risk of developing HITT increases with the duration of heparin therapy (>5 days), the type (UFH/LMWH) and dose of heparin, the indication for treatment (surgery and trauma being higher risk, exposure of tissue to platelet and coagulation activity) and the patient's gender (females being more at risk). HITT is therefore a potentially fatal immunologic complication of heparin therapy. The cardinal clinical manifestations are a fall in platelet count and an increased propensity for thromboembolism in the setting of a proximate heparin exposure, or some other noxae.

Diagnosis of HIT II involves both laboratory and clinical tests. The clinical 4T's score evaluates for the degree of thrombocytopenia, timing of platelet declines after heparin administration, presence of thrombus and the probability of other thrombocytopenia causes. This scoring system has a high negative predictive value, making it useful to exclude HITT. Moreover, this 4T scoring is important as a pre-requisite to directing laboratory tests, as an asymptomatic patient with a 4T score indicating HIT II may then have an ultrasound which finds a thrombosis, once this is established the patient needs a curative, rather than a prophylactic approach.

With the diagnosis or suspicion of HIT II or HITT, all heparins must be discontinued, and warfarin treatment needs to be reversed to prevent venous limb gangrene. All patients with HIT II require 4 weeks of anticoagulation therapy which can increase to 3 months if complicated by a thrombosis HITT. In some indications intravenous immunoglobulins can be used.

HIT II is, thus, an intensely prothrombotic disease with the unfortunate paradox that the thromboprophylactic and/or thrombosis treatment switches to an inducer of new thrombosis. It is caused by ultra-large immune complexes (ULICs) that can reach a micron in size. ULICs are composed of a polyanion such as unfractionated heparin (UFH), or other glycosaminoglycans (GAGs) or polyphosphates or DNA bound to platelet factor 4 (PF4) that is released when platelets are activated, e.g. after cardiopulmonary bypass or other forms of contact activation of coagulation. PF4 tetramers oligomerize along the UFH backbone, incorporating additional UFH molecules, additional PF4 molecules, etc. This structural modification creates a large antigen array that stabilizes an epitope on PF4 recognized by some HITT antibodies.

These antigenic complexes are capable of binding multiple anti-PF4/UFH antibodies, some of which, in turn, foster oligomerization.

Antigenic complexes also form between PF4 and glycosaminoglycans expressed by hematopoetic and vascular cells, which continue to become the target of HITT antibodies long after heparin is dissipated and metabolized.

ULICs also activate platelets through the IgG receptor FcγRIIA, releasing PF4 which perpetuates the formation of neoantigen and causing uncontrolled thrombin formation. PF4 when binding to heparin and other GAGs neutralizes them, so the anticoagulant action is hampered.

ULICs also activate neutrophils to generate DNA nets, monocytes to express tissue factor, activate complement which induces endothelium to express tissue factor and release von Willebrand factor, etc. Expression of tissue factor leads to the generation of thrombin, which amplifies activation of these cell types, exacerbating the risk of thrombosis.

Contemporary therapy involves administering maximally tolerated doses of a thrombin inhibitor or Factor Xa inhibitor or danaparoid therapy. However, a substantial proportion of patients develop new thromboembolic complication and a risk of major bleeding of up to 40% has been reported. Thus, there is a need for rationale, disease-specific interventions that act on the steps in the pathogenic process proximal to formation of thrombin that would permit lower doses of antithrombotic agents to be safe and effective. Specifically, a drug that prevents or disrupts antigen formation or prevents or disrupts immune complex formation, preferably all, would act at the most proximal step in the pathogenic process and would not only attenuate thrombin production, but would also block other deleterious effects of activating IgG-Fc receptors or activating complement.

We therefore describe herein the use of a heparin-based composition that is surprisingly effective at preventing or disrupting PF4/UFH complexes and/or preventing or disrupting the formation of ULICs.

## Statements of Invention

The present invention, in its various aspects, is as set out in the accompanying claims.

According to a first aspect of the invention there is provided an antithrombotic molecule having both antiplatelet and anticoagulant (APAC) activity comprising a human plasma protein to which there is attached, via a plurality of linker molecules, a plurality of heparin chains selected from the group comprising 4, 5, 6, 7, 8, 9, 10, 11, 12, 13, 14, 15, and 16, each chain having a MW between 10-21 KDa for use in the prevention and/or treatment of thrombocytopenia wherein said thrombocytopenia is selected from the group comprising: heparin-induced thrombocytopenia (HIT) type I; heparin-induced thrombocytopenia (HIT) type II; thrombocytopenia and thrombosis (HITT); heparin-independent thrombocytopenia aHIT; and in vaccine-induced thrombocytopenia and thrombosis (VITT).

Most ideally said thrombocytopenia is selected from the group comprising: heparin-induced thrombocytopenia (HIT) type II; and thrombocytopenia and thrombosis (HITT).

Most ideally still said thrombocytopenia is immunologically-based and selected from the group comprising: heparin-induced thrombocytopenia (HIT) type II; thrombocytopenia and thrombosis (HITT); heparin-independent thrombocytopenia aHIT; and vaccine-induced thrombocytopenia and thrombosis (VITT).

Yet more ideally said thrombocytopenia is non-immunologically-based and selected from the group comprising: heparin-induced thrombocytopenia (HIT) type I; thrombocytopenia and thrombosis (HITT); and in vaccine-induced thrombocytopenia and thrombosis (VITT).

Yet more ideally said thrombocytopenia is caused by heparin and selected from the group comprising: heparin-induced thrombocytopenia (HIT) type I; heparin-induced thrombocytopenia (HIT) type II; thrombocytopenia and thrombosis (HITT); and in vaccine-induced thrombocytopenia and thrombosis (VITT).

Without wishing to be bound by theory, we consider, APAC has properties that make it effective for use in treating thrombocytopenia. APAC has not only desirable dual antiplatelet/anticoagulant activity, but it is also a relatively small anionic molecule that competes with UFH for formation/stability of the HIT antigen and/or ULICs, making it a rational intervention in HIT type II or HITT and/or aHIT and/or VITT.

Moreover, we consider it surprising that a heparin-based composition, i.e., APAC, can be used to prevent or treat HIT type I, HIT type II or HITT; and/or VITT, diseases caused by the presence of heparin (typically, UFH/LMWH).

The manufacture and use of APAC to treat a thrombosis resulting from factors other than heparin administration, and an immunological response there against, is described in PCT/EP2015/069327 (WO/2016/030316).

Notably, HIT type II or HITT may cause either arterial and venous thrombosis and so reference herein throughout to the prevention or treatment of thrombosis refer to treatment of either an arterial and venous thrombosis.

In a yet further aspect of the invention there is provided the use of an anti-thrombotic molecule having both antiplatelet and anticoagulant (APAC) activity comprising a human plasma protein to which there is attached, via a plurality of linker molecules, a plurality of heparin chains selected from the group comprising 4, 5, 6, 7, 8, 9, 10, 11, 12, 13, 14, 15, and 16, each chain having a MW between 10-21 KDa in the manufacture of a medicament to treatment heparin-induced thrombocytopenia wherein said thrombocytopenia is selected from the group comprising: heparin-induced thrombocytopenia (HIT) type I; heparin-induced thrombocytopenia (HIT) type II; thrombocytopenia and thrombosis (HITT); heparin-independent thrombocytopenia aHIT; and in vaccine-induced thrombocytopenia and thrombosis (VITT).

In a preferred embodiment of the invention said APAC molecule has 6 or less, such as between 4, 5 or 6, heparin chains attached to said plasma protein, ideally 5 heparin chains. More preferably still, said APAC molecule has a heparin concentration of 1.1. mg/ml and said human plasma protein, which is ideally serum albumin (HSA), has a concentration of 0.87 mg/ml.

In a preferred embodiment of the invention said APAC is formulated for administration at a dose within the range including and between 0.15 $\mu$g/ml - 10 $\mu$g/ml, wherein inhibition of HIT type I, or HITT type II is almost complete at the higher end of the range. More preferably, HITT is inhibited at 0.3 $\mu$g/ml with a major inhibitory effect is seen at 1 $\mu$g/ml and above. The invention therefore comprises APAC formulated for administration, within blood or plasma, at a dose within the range including and between 0.15 $\mu$g/ml - 3 $\mu$g/ml, including all 0.1 $\mu$g/ml there between. Formulations comprising 1 - 3 $\mu$g/ml are particularly preferred. Additionally, or alternatively, formulations are preferred in the range 0.1 - 0.3 mg/kg.

In a preferred embodiment of the invention said heparin-conjugated human plasma protein is an albumin, globulin or fibrinogen, ideally it is serum albumin or alpha2-macroglobulin and more ideally human serum albumin (HSA) or human alpha2-macroglobulin. As is known generally, serum albumin is produced by the liver, is dissolved in blood plasma and is the most abundant blood protein in mammals. Serum albumin is a globular, water-soluble protein of approximate molecular weight of 66,000 Daltons. As is also known alpha2-macroglobulin ($\alpha$2M and A2M) is a large plasma protein, in fact it is the largest major non-immunoglobulin protein in plasma and is produced mainly by the liver. Alpha2-macroglobulin acts as an anti-protease and is able to inactivate a large variety of proteinases.

In yet a further preferred embodiment of the invention said plasma protein is recombinant.

In yet a further preferred embodiment of the invention said heparin is unfractionated heparin. More ideally still said heparin is of mammalian origin, ideally, human or porcine. In the instance where the plasma protein is human, and the heparin porcine or bovine heparin said APAC molecule represents a chimeric molecule.

Preferably, the heparin has a MW selected from the group comprising: 10, 11, 12, 13, 14, 15, 16, 17, 18, 19, 20 or 21 KDa, ideally 15 or 16 or 17 KDa.

In yet a further preferred embodiment of the invention said heparin is recombinant.

In yet a more preferred embodiment of the invention said linker molecule, at least when linkage of said heparin to said plasma protein is complete, is a single linker molecule that binds one molecule of heparin therefore the attachment of one linker molecule to said plasma protein results in the attachment of one molecule of heparin to said plasma protein. Thus, the stoichiometry of said linker to said heparin is 1:1. Preferably said linker is an amine linker and so links with amino groups on said heparin and plasma protein, ideally, but not exclusively, said linker conjugates with serine on the heparin chain, ideally located at the end or near the end of said chain, and ideally, but not exclusively, lysine on the plasma protein. More ideally yet said linker conjugates said heparin and plasma protein by the use of disulfide bridges. Yet more preferably, said linker is a hetero-bi-functional cross-linker such as a 3-(2-Pyridyldithio)propionic acid N-hydroxysuccinimide ester (SPDP) linker or a homo-bi-functional cross-linker such as a 3,3'-Dithiodipropionicacid di(N-hydroxysuccinimide (NHS)-ester (DTSP) linker.

SPDP (available commercially from for example from Sigma-Aldrich or Thermo Scientific Pierce) is a short-chain cross-linker used for amine-to-sulfhydryl conjugation via N-hydroxysuccinimide (NHS) -ester and pyridyldithiol reactive groups, and it forms cleavable (reducible) disulfide bonds with cysteine sulfhydryls. It is available in short chain and long chain versions. The long chain version is available in a sulfonated form and is water-soluble. We prefer to use 3-(2-Pyridyldithio)propionic acid N-hydroxysuccinimide ester. Although all the SPDPs contain an amine-reactive N-hydroxysuccinimide (NHS) ester that will react with lysine residues to form a stable amide bond and, at the other end of the linker, there is a pyridyl disulfide group that will react with sulfhydryls to form a reversible disulfide bond.

DTSP (3,3'-Dithiodipropionicacid di(N-hydroxysuccinimide (NHS)-ester), available commercially from, for example, Sigma-Aldrich or Thermo Scientific Pierce) is a short-chain cross-linker used for amine-to-amine conjugation via N-hydroxysuccinimide (NHS) ester groups. It is available in short chain and long chain versions. The long chain version is available in a sulfonated form (N-hydroxysulfosuccinimide (sulfo-NHS) ester) and is water-soluble. DTSPs contain two amine-reactive N-hydroxysuccinimide (NHS) ester groups and a disulfide bridge in the spacer arm. N-hydroxysuccinimide ester reacts with primary amine containing residues to form stable amide bonds with a cleavable disulfide bond in the linker molecule.

In a preferred embodiment said APAC had a coupling level (CL) of 5 heparins per human serum albumin (HSA) and the linker used for the coupling is SPDP.

Given the anti-thrombotic molecule/APAC has both antiplatelet and anticoagulant (APAC) activity when said APAC molecule has 6 or less, such as between 4-6, heparin chains attached to said plasma protein, said molecule functions predominantly, or to a larger extent, as an anticoagulant.

Given the antithrombotic molecule/APAC has both antiplatelet and anticoagulant (APAC) activity when said APAC molecule has between 8-16, heparin chains attached to said plasma protein when said molecule functions predominantly, or to a larger extent, as an antiplatelet/platelet inhibitor.

There is also taught herein, a method for the prevention and/or treatment of thrombocytopenia; wherein an effective amount of antithrombotic molecule is administered to an individual to be treated said antithrombotic molecule having both antiplatelet and anticoagulant (APAC) activity comprising a plasma protein to which there is attached, via a plurality of linker molecules, a plurality of heparin chains each having a MW between 10-21 KDa and further wherein the number of said heparin chains attached to said plasma protein is selected from the group comprising 4, 5, 6, 7, 8, 9, 10, 11, 12, 13, 14, 15, and 16, wherein said thrombocytopenia is selected from the group comprising: heparin-induced thrombocytopenia (HIT) type I; heparin-induced thrombocytopenia (HIT) type II; thrombocytopenia and thrombosis (HITT); heparin-independent thrombocytopenia aHIT; and in vaccine-induced thrombocytopenia and thrombosis (VITT).

There is also taught herein, said APAC is instead of heparin i.e. LMW heparin or unfractionated UFH.

In a preferred teaching of the invention said APAC is administered at a dose, in blood or plasma, within the range including and between 0.15 μg/ml - 10 μg/ml, wherein inhibition of HIT or HITT is almost complete at the higher end if the range. More preferably, HITT is inhibited at 0.3 μg/ml with a major inhibitory effect is seen at 1 μg/ml and above. The invention therefore comprises APAC formulated for administration at a dose within the range including and between 0.15 μg/ml - 3 μg/ml, including all 0.1μg/ml there between. Additionally, or alternatively, formulations are preferred in the range 0.1 - 3 mg/kg.

In the claims which follow and in the preceding description of the invention, except where the context requires otherwise due to express language or necessary implication, the word "comprises", or variations such as "comprises" or "comprising" is used in an inclusive sense, i.e. to specify the presence of the stated features but not to preclude the presence or addition of further features in various embodiments of the invention.

The present invention will now be described by way of example only with particular reference to the following figures wherein:

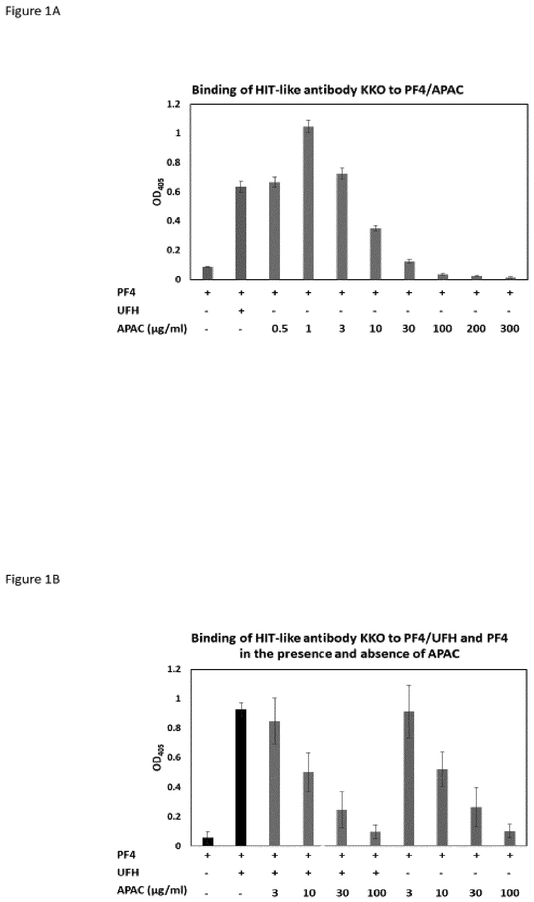

Figure 1. Shows the binding of HIT-like monoclonal Ab KKO (Arepally et al. Blood. 2000;95:1533-1540 https://www.ncbi.nlm.nih.gov/pubmed/10688805) to PF4 in the presence and absence of UFH and APAC. A, shows the binding of HIT-like monoclonal antibody KKO (a mouse monoclonal IgG[2bkappa] antibody against the complex of human PF4 and heparin) 1) to immobilized PF4 (50 μl of 5 μg/ml per well = 0.25 μg), 2) immobilized PF4 in the presence of UFH (0.1 IU/ml), and 3) immobilized PF4 in the presence of APAC (0.5, 1, 3, 10, 30, 100, 200 and 300 μg/ml; at the heparin equivalent concentration). B, shows the binding of Ab KKO 1) to immobilized PF4 (0.25 μg), 2) to immobilized PF4 in the presence of UFH (0.1 IU/ml), 3) to immobilized PF4 in the presence of both UFH (0.1 IU/ml) and APAC (3, 10, 30 and 100 μg/ml), and 4) to immobilized PF4 in the presence of APAC (3, 10, 30 and 100 μg/ml). Results are shown as mean ± standard error of the mean (SEM) of 3 independent experiments.

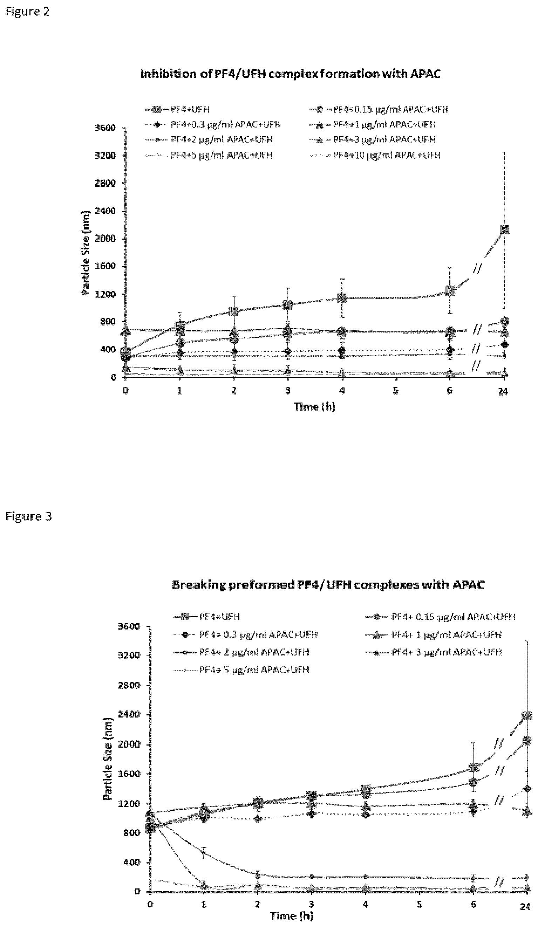

Figure 2. Shows the inhibitory effect of APAC on the formation of large antigenic PF4/UFH complexes. The size of the particle formation between PF4 (10 μg/ml) and UFH (0.1 IU/ml) in the presence and absence of APAC (0.15, 0.3, 1, 2, 3, 5 or 10 μg/ml; informed as the heparin equivalent concentration) is displayed after 0, 1, 2, 3, 4, 5, 6 and 24 hours of incubation time. Particle formation was detected by dynamic light scattering (DLS). Results are shown as mean ± standard deviation (SD) of 3 independent experiments.

Figure 3. Shows the dissociating effect of APAC on the preformed large antigenic PF4/UFH complexes. PF4/UFH complex was first formed between 10 μg/ml of PF4 and 0.2 IU/ml of UFH. The particle size of this preformed PF4/UFH complex in the presence and absence of APAC (0.15, 0.3, 1, 2, 3, 5 or 10 μg/ml; at the heparin equivalent concentration) is displayed at 0, 1, 2, 3, 4, 5, 6 and 24 hours of incubation time. Particle formation was detected by DLS. Results are shown as mean ± SD of 3 independent experiments.

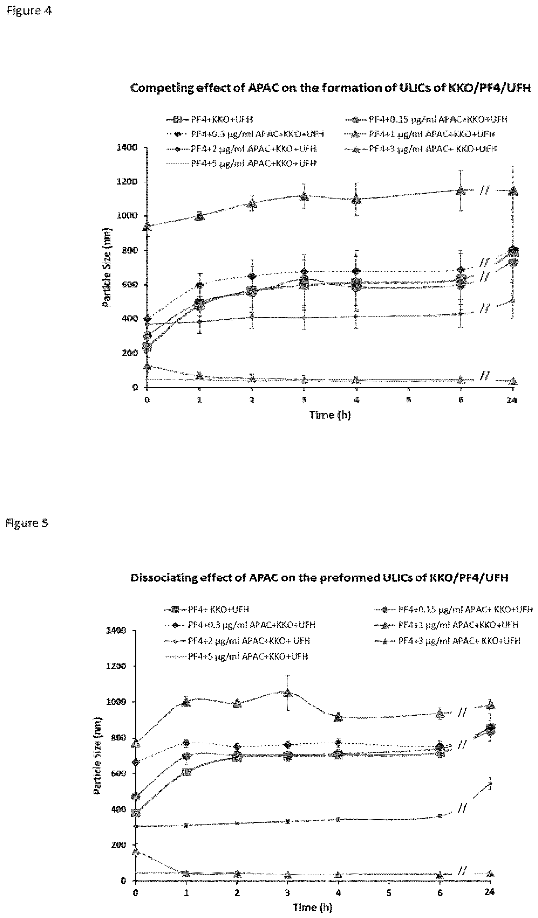

Figure 4. Shows the competing effect of APAC on the formation of ultra-large immunocomplexes (ULICs) of KKO/PF4/UFH. The size of the ULICs particle formation between PF4 (10 μg/ml), UFH (0.2 IU/ml) and HIT-like monoclonal antibody KKO (30 μg) in the presence and absence of APAC (0.15, 0.3, 1, 2, 3, 5 or 10 μg/ml; informed as the heparin equivalent concentration) is displayed at 0, 1, 2, 3, 4, 5, 6 and 24 hours of incubation time. Particle formation was detected by DLS. Results are shown as mean ± SD of 3 independent experiments

Figure 5. Shows the dissociating effect of APAC on the preformed ultra-large immunocomplexes (ULICs) of KKO/PF4/UFH. ULICs were first formed using PF4 (10 μg/ml), UFH (0.2 IU/ml) and HIT-like monoclonal antibody KKO (30 μg). The particle size of the preformed KKO/PF4/UFH complexes in the presence and absence of APAC (0.15, 0.3, 1, 2, 3, or 5 μg/ml; at the heparin equivalent concentration) is displayed at 0, 1, 2, 3, 4, 5, 6 and 24 hours of incubation time. Particle formation was detected by DLS. Results are shown as mean ± SD of 3 independent experiments.

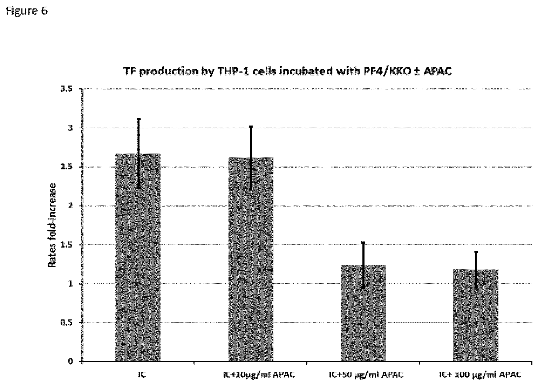

Figure 6. Shows the effect of APAC on the induction of tissue factor (TF) activity on human monocytic-like cell line (THP-1). THP-1 cells were first incubated with PF4 (10 μg/ml) and then supplemented with APAC (10, 50 or 100 μg/ml;

at the heparin equivalent concentration). Control THP-1 cells were not treated with APAC. HIT-like monoclonal antibody KKO (50 $\mu$g/ml) was included to induce formation of immunocomplexes (IC). The generation of FXa activity reflected the active TF expression in the cell suspensions. Data are depicted as the mean fold-increase of the initial velocity of FXa generation relative to THP-1 cells alone. Results are shown as mean $\pm$ SEM of 4 experiments.

## METHODS

### Conjugation

Unfractionated heparin, Hep (UFH chains were conjugated to Human Serum Albumin (HSA) through disulfide bridges created by two alternative cross-linkers and reactions routes using:

i) hetero-bi-functional cross-linker 3-(2-Pyridyldithio)propionic acid N-hydroxysuccinimide ester (SPDP). For the conjugation free amines on Ser at the Hep linker region and Lys on HSA were utilized. Hep and HSA were modified in separate reactions into sulfhydryl (-SH) - and pyridyl dithiol(-PDP)-derivatives, respectively. In the final conjugation reaction, the pyridyldithiol-group of HSA reacted with sulfhydryl group of Hep resulting in the formation of a disulphide bonded complex and the release of pyridine 2-thione.
ii) homo-bi-functional cross-linker 3,3'-Dithiodipropionicacid di(N-hydroxysuccinimide (NHS)-ester) (DTSP). For the conjugation, free amines on Ser at the Hep linker region and Lys on HSA were utilized. Hep was first modified into N-hydroxysuccinimide (NHS)-ester-derivative with the release of the first NHS-group. In the final conjugation reaction the Lys of HSA reacted with the N-hydroxysuccinimide (NHS)-ester group of the derivatized Hep, resulting in the formation of a complex with a cleavable disulfide bond in the linker region and the release of the second N-hydroxy-succinimide group.

The ratio of the above defined intermediate derivatives of HSA and Hep in the final conjugation reaction to produce Hep-HSA complexes is selected to yield the specified mean conjugation level (CL) in the final purified Hep-HSA complexes.
Hep-HSA complexes were purified by ultra/diafiltration and anion exchange chromatography using Q sepharose media (GE Healthcare, USA) or ultra/dialfiltration. At the end Hep-HSA complexes were eluted into phosphate buffered saline (PBS) with pH 7.4-7.5. Complexes were named as APAC- with a suffix extension designating the mean conjugation level of Hep chains to HSA. Accordingly, reference herein to a plurality of heparin chains conjugated to a human plasma protein selected from the group comprising 4, 5, 6, 7, 8, 9, 10, 11, 12, 13, 14, 15, and 16, is reference to a mean conjugation level.
The general formula for APAC complexes that exemplify the invention is

$$(\text{Hep-NH-CO-CH}_2\text{-CH}_2\text{-S-S-CH}_2\text{-CH}_2\text{-CO-NH})\text{n-HSA}$$

where the average number of unfractionated heparin chains coupled to HSA is defined as n.
The mean conjugation level (CL) of Hep to HSA was determined using the concentration of Hep and HSA and their average molecular weights with the following equations:

$$\text{mol of Hep} = \text{Hep [C]/mean Hep MW}$$

$$\text{mol of HSA} = \text{HSA [C]/HSA MW}$$

$$\text{CL} = \text{mol of Hep/mol of HSA}$$

$$\text{Hep MW} = 15800 \text{ or } 17000$$

$$\text{HSA MW} = 66472$$

### Binding of HIT-like monoclonal Ab KKO to PF4 in the presence and absence of UFH and APAC

Immulon 4 HBX plates (Thermo Scientific, Waltham, MA, USA) were first coated with PF4 (50 $\mu$l of 5 $\mu$g/ml in phosbate buffered saline [PBS]). In the experiment A, wells were supplemented with APAC at final concentration of 0.5, 1, 3, 10, 30, 100, 200 and 300 μg/ml. In the experiment B), APAC was supplemented at final concentration of 3, 10, 30, 100, 200 and 300 μg/ml either alone or together with constant concentration of UFH (0.1 IU/ml). PF4 alone and PF4 supplemented with UFH (0.1 IU/ml) were used as controls. Plates were incubated overnight at room temperature (RT). The next day, the wells were washed 4 times with 180 μl of PBS and the unspecific binding was blocked with 1% bovine serum albumin (BSA) in PBS for 1 hour at RT. Next, wells were supplemented with HIT-like monoclonal antibody KKO at 100 μg/ml (in 1% BSA/PBS) for 30 min at 37°C after which wells were washed 4 times with 180 μl of PBS/0.1% Tween-20. Wells were incubated 30 min with Horse radish peroxidase (HRP) conjugated Goat Anti-Mouse IgG (Fc) 1:3000 in 1% BSA/PBS was used as the secondary Ab (Bethyl Laboratories, Montgomery, TX, US). The wells were further washed 4 times with 180 μl PBS/0.1% Tween-20 and HRP substrate, 2,2'-Azino-bis(3-ethylbenzothiazoline-6-sulfonic acid) diammonium salt (Roche diagnostics, Mannheim, Germany) was added at 100 μl/well at RT to detect the secondary Ab at 405 nm and 490 nm on a BioTek Synergy 2 plate reader (BioTek Instruments Inc., Winooski, VT, USA). Results are calculated as mean $\pm$ SEM of 3 independent experiments.

### Effect of APAC on formation of large antigenic PF4/UFH complexes

PF4 (10 μg/ml) was supplemented either with UFH (Hep; 0.2 IU/ml) alone, or with both UFH (Hep; 0.2 IU/ml) and APAC at increasing concentrations (0.15, 0.3, 1, 2, 3, 5 or 10 μg/ml). Particle size was measured by dynamic light scattering (DLS) immediately after addition of UFH and/or APAC and after 1, 2, 3, 4, 6, and 24 hours of incubation. Results are calculated as mean $\pm$ SD of 3 independent experiments.

### Effect of APAC on dissociation of preformed large antigenic complexes

PF4 (10 μg/ml) was supplemented with UFH (0.2 IU/ml) and pre-incubated for 30 min at room temperature after which APAC was added at the increasing concentrations of 0.15, 0.3, 0.5, 1, 2, 3, and 5 μg/ml. The size of the formed particles was measured by DLS immediately and after 1, 2, 3, 4, 6, and 24 hours of incubation. Results are calculated as mean $\pm$ SD of 3 independent experiments.

### Effect of APAC on formation of ultra large immunocomplexes

APAC (0.15, 0.3, 1, 2, 3, or 5 μg/ml), PF4 (10 μg/ml), UFH (0.2 IU/ml) and HITT-like monoclonal antibody KKO (30 μg) were incubated together and the size of the formed ultra large immunocomplexes (ULICs) was measured by DLS immediately, and after 1, 2, 3, 4, 6, and 24 hours of incubation. Results are calculated as mean $\pm$ SD of 3 independent experiments.

### Effect of APAC on disruption of preformed ultra large immunocomplexes

PF4 (10 μg/ml) was first incubated with HIT-like monoclonal Ab KKO for 5 min at RT after which, UFH (0.2 IU/ml) was added for additional 5 min to form PF4/KKO/UFH complexes. These pre-formed PF4/KKO/UFH complexes were then supplemented with APAC at 0.15, 0.3, 1, 2, 3, or 5 μg/ml. The size of the formed particles was measured by DLS immediately, and after 1, 2, 3, 4, 6, and 24 hours of incubation. Results are calculated as mean $\pm$ SD of 3 independent experiments.

### Effect of APAC on the induction of FXa activity by a monocytic cell line

Tissue factor, TF production by THP-1 cells incubated with PF4/KKO $\pm$ APAC. This experiment was designed to determine whether APAC would prevent the generation of tissue factor (TF) activity on human monocyte-like cells by PF4 and HIT-like monoclonal antibody KKO.

THP-1 cells (a human acute leukemia monocytic cell line) were cultured in Roswell Park Memorial Institute (RPMI) 1640 medium supplemented with 10% fetal bovine serum (FBS), 4.5 mg/ml glucose, 1 mM sodium pyruvate, 2 mM L-glutamine, 100 U/mL penicillin, 100 μg/ml streptomycin, and 0.25 μg/ml amphotericin B, at 37°C and under 5% $CO_2$. THP-1 cells were plated on a 96-well plate at $10^5$ cells per well in 100 μl of RPMI-1640 supplemented with 5 % FBS. THP-1 cells were incubated at 37°C first with PF4 (10 μg/ml) for 5 min, and then for additional 30 min with APAC at final concentration of 10, 50 or 100 μg/ml. Control THP-1 cells were without APAC. In the third step, all cells were supplemented with HIT-like monoclonal Ab KKO (50 μg/ml) and incubated further overnight. Binding of KKO to cell-associated glycosaminoglycans substituted for exogenous UFH. The next day cells were washed to remove unbound ligands. FXa activity was measured using a chromogenic assay in a flat bottom 96-well plate where an aliquot (10 μl) of cell suspension was added to a mixture of Factor VIIa (0.5 nM) and Factor X (160 nm) in 20 mM Tris buffer, pH 7.4 containing 100 mM NaCl and 10 mM CaCl2 for 30 min at 37°C under 5% $CO_2$. An activated coagulation factor FXa chromogenic substrate (0.4 mM) was added and the optical density at 405 nm was read in a kinetic mode (one read per minute) for 30 min at 37°C. The amount of FXa generated over the first 10 min was calculated relative to a standard curve using purified reagents.

The mean MW for the Hep polymer is based on the information obtained from the heparin manufacturer. HSA MW is based on ALBU_HUMAN, P02768 from UniProtKB/Swiss-Prot, isoform 1 without signal- and propeptide.

Statistics. All data are mean $\pm$ SD or SEM and analyzed by SPSS for Windows, version 15.0 (SPSS Inc, Chicago, IL). For two-group comparison, non-parametric Mann-Whitney U test and parametric Student's t-test were applied. For multiple-group comparison, non-parametric Kruskal-Wallis test with the Dunn post hoc test and parametric ANOVA with Dunnett's correction were applied. P<0.05 was regarded as statistically significant.

## Experimental Procedure

Our first objective was to determine whether APAC formed antigenic complexes with PF4 and/or whether it reduced the antigenicity of PF4/UFH complexes, defined by binding of a HIT-like antibody. Once we identified a concentration of APAC that interfered with antigenicity, our second objective was to examine the impact on the size of the large pathogenic complexes that cause HIT or HITT. Because we have found that ULICs are stable for over 24 hours, we assumed that the antigenic complex and the immune complex undergo a succession of changes over time that make it progressively more difficult to effect change. Therefore, our objective was to perform a series of experiments addressing the presumed sequence of events in the pathogenesis of HIT by asking the following 4 questions, each addressed in turn:

1) Can APAC prevent formation of PF4/UFH complexes?
2) Can APAC disrupt PF4/UFH complexes?
3) Can APAC prevent formation of ULICs? and
4) Can APAC disrupt preformed ULICs?

To do so we used a well described murine monoclonal anti-PF4/UFH antibody called KKO, and we employed dynamic light scattering (DLS) to measure the size of complexes in solution. Lastly, we asked whether APAC would inhibit the ability of ULICs to generate active coagulation factor, FXa activity on a monocytic cell line.

## Results

Note: In all experiments, the data are presented as mean $\pm$ SD of at least 3 independent experiments (Fig. 2-5) or mean $\pm$ SEM from 3 (Fig. 1) or 4 experiments (Fig. 6).

### A. Effect of APAC on binding of the HIT-like monoclonal antibody KKO.

The data shown in Fig.1A are from ELISAs to measure the binding of the murine monoclonal HIT-like antibody to PF4/APAC. The results show that KKO binds to PF4/APAC starting at the lowest concentration tested (0.5 $\mu$g/ml) (see Figure 1A). However, binding of KKO is lower in a dose dependent manner at higher, likely therapeutic concentrations of APAC based on subsequent results. This is the same pattern as is observed at supraoptimal concentrations of heparin attributed to formation of smaller complexes with PF4.

### The effect of APAC added to PF4/UFH is shown in Figure 1B, left side.

There is a small increase in KKO binding to PF4/UFH at a concentration of APAC of 1 $\mu$g/ml, which is equivalent to binding to PF4/APAC alone, i.e. in the absence of UFH (data not shown). However, the most important effect is a dose-dependent decrease in KKO binding at all higher concentrations of APAC. Comparison of the right and left slides in Figure 1B makes it likely that APAC dissociated large PF4/UFH complexes and binding is to, what we will propose will be small, PF4/APAC complexes. These results led to ask whether binding of PF4 to APAC generates large "pathogenic" complexes. We thought that the small size of APAC makes it unlikely to foster oligomerization of PF4 in the manner seen with UFH, and this supposition was confirmed in the experiments that are described below.

### B. Effect of APAC on formation of large antigenic PF4/UFH complexes.

In these and the DLS experiments that follow, PF4 (10 $\mu$g/ml) was incubated with the indicated concentration of APAC and UFH (0.2 IU/ml) as the standard starting condition. Particle size (ordinate) was measured by DLS immediately and 1, 2, 3, 4, 6, and 24 hours later (abscissa).

The red line in Figure 2 shows the absence of APAC, i.e. this is the sizes of the complexes formed between PF4 and UFH, which continue to increase in time over 24 hours incubation. In general, there is an inverse dose-dependent relationship between the concentration of APAC and size of the particles. The seemingly anomalous early result at 1 μg/ml APAC matches results of the ELISA and may represent a combination of PF4/APAC and PF4/UFH complexes or incorporation of APAC into PF4/UFH complexes. The results show that growth of complexes during 24 hours of incubation is inhibited by APAC at concentrations as low as 0.15 μg/ml and inhibition is almost complete at 3 μg/ml.

### C. Effect of APAC on dissociation of preformed large antigenic complexes.

In the set of experiments shown in Figure 3, PF4 was pre-incubated with unfractionated heparin (UFH) for 30 min at room temperature. APAC was then added at the indicated concentrations. The results show that at the lowest concentration of APAC, there is no effect on the size of the antigenic complexes. A decrease in size begins at 0.3 μg/ml and no complexes >20 nm in size is evident at the higher concentrations. A major inhibitory effect is seen at 1 μg/ml.

### D. Effect of APAC on formation of ULICs.

In the experiments shown in Figure 4, APAC was added along with PF4 (10 μg/ml), UFH (0.2 IU/ml) and KKO (30 μg) and the size of the complexes (ordinate) over time (abscissa) was measured. The data show that at low doses of APAC, immune complex formation is enhanced. This is consistent with data in previous modes showing enhanced antibody binding at formation of UFH-PF4 antigenic complexes at these concentrations. At higher concentrations, APAC totally prevented formation of ultra-large immune complexes (ULICs), again consistent with its capacity to prevent and to disrupt antigen formation. A major inhibitory effect is seen at 3 μg/ml.

### E. Disruption of preformed ULICs.

This is the most stringent test, i.e. breaking up the large and stable pre-formed PF4/KKO/heparin complexes. Here, PF4 (10 μg/ml) was incubated with KKO for 5 min at RT. UFH (0.2 IU/ml) was added for 5 min at RT. Then APAC was added at increasing concentrations (0 to 5 μg/ml). The results are shown in Figure 5. As we observed in all previous experimental conformations, low doses of APAC increased the size of the complexes. However, preformed complexes were totally disrupted at higher concentrations of APAC. A major inhibitory effect is seen at 3 μg/ml.

### F. Effect of APAC on the induction of FXa activity by a monocytic cell line.

This experiment was designed to determine whether APAC would prevent the induction of tissue factor activity on THP-1 monocytic cells by PF4 and KKO. THP-1 cells were incubated with PF4 with/without APAC as follows. The order of addition was PF4 (10 μg/ml, 5 min incubation) followed by APAC (30 min) and then KKO (50 μg/ml), all at 37°C. Binding of KKO to cell-associated glycosaminoglycans substituted for exogenous UFH. After further incubation, the cells were washed to remove unbound ligands. As the measure of TF expression, an aliquot of cell suspension was detected for the amount of FXa generated. Results shown in Figure 6 are the mean ± SEM of 4 experiments, each conducted in quadruplicate wells. The results show that the higher concentrations of APAC inhibited the generation of FXa by this monocytic cell line that had been stimulated by HITT immune complexes. These results are in line with all the previous sets of experiments looking at formation/dissolution of PF4/UFH and PF4/UFH/KKO complexes.

### Conclusions

These experiments support the concept that APAC provides a new approach to the treatment of HIT type II and/or HITT by combining antithrombotic activity with the capacity to interfere with ULIC formation and stability, one of the most proximal steps in the pathogenic process.

There is a successive increase in the concentration of APAC required to prevent antigen formation < dissociate antigen < prevent=dissociate immune complexes. In practical terms, administration of APAC instead of UFH would, in theory, prevent HIT from developing while higher concentrations can be used to interrupt the cycle of ULIC formation, cell activation, release of PF4 and thrombin and the feedforward prothrombotic loop that develops in these patients.

In other words, APAC should be used instead of heparin

1) Prophylactically, when there is a great suspicion of developing HIT, such as a previous history or when there is an increased risk of HIT, such as in connection with a trauma or surgical, e.g. cardiovascular, intervention and/or

2) when HIT type II or HITT has occurred then heparin needs to be stopped and APAC should replace heparin.

**Claims**

1. An antithrombotic molecule having both antiplatelet and anticoagulant (APAC) activity comprising a human plasma protein to which there is attached, via a plurality of linker molecules, a plurality of heparin chains selected from the group comprising 4, 5, 6, 7, 8, 9, 10, 11, 12, 13, 14, 15, and 16, each chain having a MW in the range 10-21 KDa for use in the prevention and/or treatment of thrombocytopenia;
wherein said thrombocytopenia is selected from the group comprising, including any combination thereof: heparin-induced thrombocytopenia (HIT) type I; heparin-induced thrombocytopenia (HIT) type II; heparin-induced thrombocytopenia and thrombosis (HITT); heparin-independent thrombocytopenia aHIT; and in vaccine-induced thrombocytopenia and thrombosis (VITT).

2. The antithrombotic molecule for use in the prevention and/or treatment of thrombocytopenia according to claim 1 wherein said thrombocytopenia is selected from the group comprising: heparin-induced thrombocytopenia (HIT) type II; and heparin-induced thrombocytopenia and thrombosis (HITT).

3. The antithrombotic molecule for use in the prevention and/or treatment of thrombocytopenia according to claim 1 wherein said thrombocytopenia is immunologically-based and selected from the group comprising: heparin-induced thrombocytopenia (HIT) type II; heparin-induced thrombocytopenia and thrombosis (HITT); heparin-independent thrombocytopenia aHIT; and in vaccine-induced thrombocytopenia and thrombosis (VITT).

4. The antithrombotic molecule for use in the prevention and/or treatment of thrombocytopenia according to claim 1 wherein said thrombocytopenia is non-immunologically-based and selected from the group comprising: heparin-induced thrombocytopenia (HIT) type I; heparin-induced thrombocytopenia and thrombosis (HITT); and in vaccine-induced thrombocytopenia and thrombosis (VITT).

5. The antithrombotic molecule for use in the prevention and/or treatment of thrombocytopenia according to claim 1 wherein said thrombocytopenia is caused by heparin and selected from the group comprising: heparin-induced thrombocytopenia (HIT) type I; heparin-induced thrombocytopenia (HIT) type II; thrombocytopenia and thrombosis (HITT); and in vaccine-induced thrombocytopenia and thrombosis (VITT).

6. The antithrombotic molecule for use in the prevention and/or treatment of thrombocytopenia according to any one of claims 1 - 5 wherein said APAC molecule has 4, 5 or 6, heparin chains attached to said plasma protein.

7. The antithrombotic molecule for use in the prevention and/or treatment of thrombocytopenia according to any one of claims 1 - 6 wherein said APAC molecule has 5 heparin chains attached to said plasma protein.

8. The antithrombotic molecule for use in the prevention and/or treatment of thrombocytopenia according to any one of claims 1 - 7 wherein said APAC is formulated for administration at a dose, in blood or plasma, within the range 0.15 $\mu$g/ml - 10 $\mu$g/ml, including all 0.1 $\mu$g/ml there between.

9. The antithrombotic molecule for use in the prevention and/or treatment of thrombocytopenia according to claim 8 wherein said APAC is formulated for administration at a dose within the range 1 $\mu$g/ml - 3 $\mu$g/ml, including all 0.1 $\mu$g/ml there between.

10. The antithrombotic molecule for use in the prevention and/or treatment of thrombocytopenia according to any one of claims 1 - 7 wherein said APAC is formulated for administration at a dose within the range 0.1 - 0.3 mg/kg.

11. The antithrombotic molecule for use in the prevention and/or treatment of thrombocytopenia according to any one of the preceding claims wherein said human plasma protein is selected from the group comprising or consisting of: albumin, globulin or fibrinogen.

12. The antithrombotic molecule for use in the prevention and/or treatment of thrombocytopenia according to claim 11 wherein said human plasma protein is serum albumin or alpha2-macroglobulin.

13. The antithrombotic molecule for use in the prevention and/or treatment of thrombocytopenia according to any one of the preceding claims wherein said human plasma protein is recombinant.

14. The antithrombotic molecule for use in the prevention and/or treatment of thrombocytopenia according to any one of the preceding claims wherein said heparin is unfractionated heparin.

15. The antithrombotic molecule for use in the prevention and/or treatment of thrombocytopenia according to any one of the preceding claims wherein said heparin has a MW of 15 or 16 or 17 KDa.

16. The antithrombotic molecule for use in the prevention and/or treatment of thrombocytopenia according to any one of the preceding claims wherein said heparin is recombinant.

17. The antithrombotic molecule for use in the prevention and/or treatment of thrombocytopenia according to any one of the preceding claims wherein each linker molecule binds one molecule of heparin to said human plasma protein.

18. The antithrombotic molecule for use in the prevention and/or treatment of thrombocytopenia according to any one of the preceding claims wherein said linker is an amine linker and so links with amino groups on said heparin and plasma protein.

19. The antithrombotic molecule for use in the prevention and/or treatment of thrombocytopenia according to any one of the preceding claims wherein said linker conjugates with serine on the heparin chain and a lysine on the plasma protein.

20. The antithrombotic molecule for use in the prevention and/or treatment of thrombocytopenia according to any one of the preceding claims wherein said linker is a hetero-bi-functional cross-linker such as a 3-(2-Pyridyldithio)propionic acid N-hydroxysuccinimide ester (SPDP) linker or a homo-bi-functional cross-linker such as a 3,3'-Dithiodipropionicacid di(N-hydroxysuccinimide (NHS)-ester) (DTSP) linker.

21. The antithrombotic molecule for use in the prevention and/or treatment of thrombocytopenia according to any one of the preceding claims wherein said APAC has a coupling level (CL) of 5 heparins per human serum albumin (HSA) and the linker used for the coupling is SPDP.


**Patentansprüche**

1. Antithrombisches Molekül sowohl mit Antiplättchen- als auch mit antikoagulativer (APAC) Aktivität, umfassend ein Humanplasmaprotein, an das über eine Vielzahl von Linker-Molekülen eine Vielzahl von Heparinketten, ausgewählt aus der Gruppe umfassend 4, 5, 6, 7, 8, 9, 10, 11, 12, 13, 14, 15 und 16, angelagert ist, wobei jede Kette ein Molekulargewicht, MW, im Bereich von 10-21 kDa aufweist, zur Verwendung bei der Prävention und/oder Behandlung von Thrombozytopenie;
wobei die Thrombozytopenie ausgewählt ist aus der Gruppe umfassend, einschließlich einer beliebigen Kombination davon: Heparin-induzierte Thrombozytopenie (HIT) Typ I; Heparin-induzierte Thrombozytopenie (HIT) Typ II; Heparin-induzierte Thrombozytopenie und Thrombose (HITT); Heparin-unabhängige Thrombozytopenie aHIT; und bei Impfstoff-induzierte Thrombozytopenie und Thrombose (VITT).

2. Antithrombisches Molekül zur Verwendung bei der Prävention und/oder Behandlung von Thrombozytopenie nach Anspruch 1, wobei die Thrombozytopenie ausgewählt ist aus der Gruppe umfassend: Heparin-induzierte Thrombozytopenie (HIT) Typ II; und Heparin-induzierte Thrombozytopenie und Thrombose (HITT).

3. Antithrombisches Molekül zur Verwendung bei der Prävention und/oder Behandlung von Thrombozytopenie nach Anspruch 1, wobei die Thrombozytopenie immunologisch basiert ist und ausgewählt ist aus der Gruppe umfassend: Heparin-induzierte Thrombozytopenie (HIT) Typ II; Heparin-induzierte Thrombozytopenie und Thrombose (HITT); Heparin-unabhängige Thrombozytopenie aHIT; und bei Impfstoff-induzierte Thrombozytopenie und Thrombose (VITT).

4. Antithrombisches Molekül zur Verwendung bei der Prävention und/oder Behandlung von Thrombozytopenie nach Anspruch 1, wobei die Thrombozytopenie nicht immunologisch basiert ist und ausgewählt ist aus der Gruppe umfassend: Heparin-induzierte Thrombozytopenie (HIT) Typ I; Heparin-induzierte Thrombozytopenie und Thrombose (HITT); und bei Impfstoff-induzierte Thrombozytopenie und Thrombose (VITT).

5. Antithrombisches Molekül zur Verwendung bei der Prävention und/oder Behandlung von Thrombozytopenie nach Anspruch 1, wobei die Thrombozytopenie durch Heparin verursacht wird und ausgewählt ist aus der Gruppe umfassend: Heparin-induzierte Thrombozytopenie (HIT) Typ I; Heparin-induzierte Thrombozytopenie (HIT) Typ II; Thrombozytopenie und Thrombose (HITT); und bei Impfstoff-induzierte Thrombozytopenie und Thrombose (VITT).

6. Antithrombisches Molekül zur Verwendung bei der Prävention und/oder Behandlung von Thrombozytopenie nach einem der Ansprüche 1 - 5, wobei das APAC-Molekül 4, 5 oder 6 Heparinketten aufweist, die an das Plasmaprotein angelagert sind.

7. Antithrombisches Molekül zur Verwendung bei der Prävention und/oder Behandlung von Thrombozytopenie nach einem der Ansprüche 1 - 6, wobei das APAC-Molekül 5 Heparinketten aufweist, die an das Plasmaprotein angelagert sind.

8. Antithrombisches Molekül zur Verwendung bei der Prävention und/oder Behandlung von Thrombozytopenie nach einem der Ansprüche 1 - 7, wobei das APAC zur Verabreichung in einer Dosis, in Blut oder Plasma, innerhalb des Bereichs von 0,15 $\mu$g/ml - 10 $\mu$g/ml, einschließlich aller 0,1 $\mu$g/ml dazwischen, formuliert ist.

9. Antithrombisches Molekül zur Verwendung bei der Prävention und/oder Behandlung von Thrombozytopenie nach Anspruch 8, wobei das APAC zur Verabreichung in einer Dosis innerhalb des Bereichs von 1 $\mu$g/ml - 3 $\mu$g/ml, einschließlich aller 0,1 $\mu$g/ml dazwischen, formuliert ist.

10. Antithrombisches Molekül zur Verwendung bei der Prävention und/oder Behandlung von Thrombozytopenie nach einem der Ansprüche 1 - 7, wobei das APAC zur Verabreichung in einer Dosis innerhalb des Bereichs von 0,1 - 0,3 mg/kg formuliert ist.

11. Antithrombisches Molekül zur Verwendung bei der Prävention und/oder Behandlung von Thrombozytopenie nach einem der vorhergehenden Ansprüche, wobei das Humanplasmaprotein ausgewählt ist aus der Gruppe umfassend oder bestehend aus: Albumin, Globulin oder Fibrinogen.

12. Antithrombisches Molekül zur Verwendung bei der Prävention und/oder Behandlung von Thrombozytopenie nach Anspruch 11, wobei das Humanplasmaprotein Serumalbumin oder Alpha2-Makroglobulin ist.

13. Antithrombisches Molekül zur Verwendung bei der Prävention und/oder Behandlung von Thrombozytopenie nach einem der vorhergehenden Ansprüche, wobei das Humanplasmaprotein rekombinant ist.

14. Antithrombisches Molekül zur Verwendung bei der Prävention und/oder Behandlung von Thrombozytopenie nach einem der vorhergehenden Ansprüche, wobei das Heparin unfraktioniertes Heparin ist.

15. Antithrombisches Molekül zur Verwendung bei der Prävention und/oder Behandlung von Thrombozytopenie nach einem der vorhergehenden Ansprüche, wobei das Heparin ein MW von 15 oder 16 oder 17 kDa aufweist.

16. Antithrombisches Molekül zur Verwendung bei der Prävention und/oder Behandlung von Thrombozytopenie nach einem der vorhergehenden Ansprüche, wobei das Heparin rekombinant ist.

17. Antithrombisches Molekül zur Verwendung bei der Prävention und/oder Behandlung von Thrombozytopenie nach einem der vorhergehenden Ansprüche, wobei jedes Linker-Molekül ein Molekül Heparin an das Humanplasmaprotein bindet.

18. Antithrombisches Molekül zur Verwendung bei der Prävention und/oder Behandlung von Thrombozytopenie nach einem der vorhergehenden Ansprüche, wobei der Linker ein Amin-Linker ist und daher mit Aminogruppen an das Heparin- und Plasmaprotein bindet.

19. Antithrombisches Molekül zur Verwendung bei der Prävention und/oder Behandlung von Thrombozytopenie nach einem der vorhergehenden Ansprüche, wobei der Linker mit Serin an der Heparinkette und einem Lysin am Plasmaprotein konjugiert.

20. Antithrombisches Molekül zur Verwendung bei der Prävention und/oder Behandlung von Thrombozytopenie nach einem der vorhergehenden Ansprüche, wobei der Linker ein heterobifunktioneller Vernetzer, wie ein 3-(2-Pyridyldithio)propionsäure-N-hydroxysuccinimidester (SPDP)-Linker, oder ein homo-bifunktioneller Vernetzer, wie ein 3,3'-Dithiodipropionsäuredi-(N-hydroxysuccinimid (NHS)-ester) (DTSP)-Linker, ist.

21. Antithrombisches Molekül zur Verwendung bei der Prävention und/oder Behandlung von Thrombozytopenie nach einem der vorhergehenden Ansprüche, wobei der APAC einen Kopplungsgrad (CL) von 5 Heparinen pro Humanserumalbumin (HSA) aufweist und der für die Kopplung verwendete Linker SPDP ist.

**Revendications**

1. Molécule antithrombotique comportant une activité à la fois antiplaquettaire et anticoagulante (APAC) comprenant une protéine plasmatique humaine à laquelle est fixée, via une pluralité de molécules de liaison, une pluralité de chaînes d'héparine choisies dans le groupe comprenant 4, 5, 6, 7, 8, 9, 10, 11, 12, 13, 14, 15 et 16, chaque chaîne comportant un PM compris entre 10 et 21 KDa destiné à être utilisé dans la prévention et/ou le traitement de la thrombocytopénie ;
   ladite thrombocytopénie étant choisie dans le groupe comprenant, y compris toute combinaison de celles-ci : la thrombocytopénie induite par l'héparine (TIH) de type I ; la thrombocytopénie induite par l'héparine (TIH) de type II ; la thrombocytopénie et la thrombose induites par l'héparine (TTIH) ; la thrombocytopénie indépendante de l'héparine aTIH ; et la thrombocytopénie et la thrombose induites par le vaccin (TTIV).

2. Molécule antithrombotique destinée à être utilisée dans la prévention et/ou le traitement de la thrombocytopénie selon la revendication 1, ladite thrombocytopénie étant choisie dans le groupe comprenant : la thrombocytopénie induite par l'héparine (TIH) de type II ; et la thrombocytopénie et la thrombose induites par l'héparine (TTIH).

3. Molécule antithrombotique destinée à être utilisée dans la prévention et/ou le traitement de la thrombocytopénie selon la revendication 1, ladite thrombocytopénie étant à base immunologique et choisie dans le groupe comprenant : la thrombocytopénie induite par l'héparine (TIH) de type II ; la thrombocytopénie et la thrombose induites par l'héparine (TTIH) ; la thrombocytopénie indépendante de l'héparine aTIH ; et la thrombocytopénie et la thrombose induites par le vaccin (TTIV).

4. Molécule antithrombotique destinée à être utilisée dans la prévention et/ou le traitement de la thrombocytopénie selon la revendication 1, ladite thrombocytopénie étant à base non immunologique et choisie dans le groupe comprenant : la thrombocytopénie induite par l'héparine (TIH) de type I ; la thrombocytopénie et la thrombose induites par l'héparine (TTIH) ; et la thrombocytopénie et la thrombose induites par le vaccin (TTIV).

5. Molécule antithrombotique destinée à être utilisée dans la prévention et/ou le traitement de la thrombocytopénie selon la revendication 1, ladite thrombocytopénie étant causée par l'héparine et choisie dans le groupe comprenant : la thrombocytopénie induite par l'héparine (TIH) de type I ; la thrombocytopénie induite par l'héparine (TIH) de type II ; la thrombocytopénie et la thrombose (TTIH) ; et la thrombocytopénie et la thrombose induites par le vaccin (TTIV).

6. Molécule antithrombotique destinée à être utilisée dans la prévention et/ou le traitement de la thrombocytopénie selon l'une quelconque des revendications 1 à 5, ladite molécule APAC comportant 4, 5 ou 6 chaînes d'héparine fixées à ladite protéine plasmatique.

7. Molécule antithrombotique destinée à être utilisée dans la prévention et/ou le traitement de la thrombocytopénie selon l'une quelconque des revendications 1 à 6, dans laquelle ladite molécule APAC a 5 chaînes d'héparine fixées à ladite protéine plasmatique.

8. Molécule antithrombotique destinée à être utilisée dans la prévention et/ou le traitement de la thrombocytopénie selon l'une quelconque des revendications 1 à 7, dans laquelle ladite APAC est formulée pour une administration à une dose, dans le sang ou le plasma, comprise entre 0,15 $\mu$g/ml et 10 $\mu$g/ml, y compris tous les 0,1 $\mu$g/ml entre ces valeurs.

9. Molécule antithrombotique destinée à être utilisée dans la prévention et/ou le traitement de la thrombocytopénie selon la revendication 8, dans laquelle ladite APAC est formulée pour une administration à une dose comprise entre 1 $\mu$g/ml et 3 $\mu$g/ml, y compris tous les 0,1 $\mu$g/ml entre ces valeurs.

10. Molécule antithrombotique destinée à être utilisée dans la prévention et/ou le traitement de la thrombocytopénie selon l'une quelconque des revendications 1 à 7, dans laquelle ladite APAC est formulée pour une administration à une dose comprise entre 0,1 et 0,3 mg/kg.

**11.** Molécule antithrombotique destinée à être utilisée dans la prévention et/ou le traitement de la thrombocytopénie selon l'une quelconque des revendications précédentes, dans laquelle ladite protéine plasmatique humaine est choisie dans le groupe comprenant ou constitué de : albumine, globuline ou fibrinogène.

**12.** Molécule antithrombotique destinée à être utilisée dans la prévention et/ou le traitement de la thrombocytopénie selon la revendication 11, dans laquelle ladite protéine plasmatique humaine est l'albumine sérique ou l'alpha2-macroglobuline.

**13.** Molécule antithrombotique destinée à être utilisée dans la prévention et/ou le traitement de la thrombocytopénie selon l'une quelconque des revendications précédentes, dans laquelle ladite protéine plasmatique humaine est recombinante.

**14.** Molécule antithrombotique destinée à être utilisée dans la prévention et/ou le traitement de la thrombocytopénie selon l'une quelconque des revendications précédentes, dans laquelle ladite héparine est de l'héparine non fractionnée.

**15.** Molécule antithrombotique destinée à être utilisée dans la prévention et/ou le traitement de la thrombocytopénie selon l'une quelconque des revendications précédentes, dans laquelle ladite héparine comporte un poids moléculaire de 15 ou 16 ou 17 KDa.

**16.** Molécule antithrombotique destinée à être utilisée dans la prévention et/ou le traitement de la thrombocytopénie selon l'une quelconque des revendications précédentes, dans laquelle ladite héparine est recombinante.

**17.** Molécule antithrombotique destinée à être utilisée dans la prévention et/ou le traitement de la thrombocytopénie selon l'une quelconque des revendications précédentes, dans laquelle chaque molécule de liaison lie une molécule d'héparine à ladite protéine plasmatique humaine.

**18.** Molécule antithrombotique destinée à être utilisée dans la prévention et/ou le traitement de la thrombocytopénie selon l'une quelconque des revendications précédentes, dans laquelle ledit lieur est un lieur amine et se lie ainsi à des groupes amino sur ladite héparine et ladite protéine plasmatique.

**19.** Molécule antithrombotique destinée à être utilisée dans la prévention et/ou le traitement de la thrombocytopénie selon l'une quelconque des revendications précédentes, dans laquelle ledit lieur se conjugue avec de la sérine sur la chaîne héparine et une lysine sur la protéine plasmatique.

**20.** Molécule antithrombotique destinée à être utilisée dans la prévention et/ou le traitement de la thrombocytopénie selon l'une quelconque des revendications précédentes, dans laquelle ledit lieur est un agent de réticulation hétéro-bi-fonctionnel tel qu'un lieur ester N-hydroxysuccinimide de l'acide 3-(2-pyridyldithio)propionique (SPDP) ou un agent de réticulation homo-bi-fonctionnel tel qu'un lieur di(N-hydroxysuccinimide (NHS)-ester) de l'acide 3,3'-dithiodipropionique (DTSP).

**21.** Molécule antithrombotique destinée à être utilisée dans la prévention et/ou le traitement de la thrombocytopénie selon l'une quelconque des revendications précédentes dans laquelle ladite APAC a un niveau de couplage (CL) de 5 héparines par sérum albumine humaine (HSA) et le lieur utilisé pour le couplage est SPDP.

Figure 1A

Figure 1B

Figure 2

Inhibition of PF4/UFH complex formation with APAC

Figure 3

Breaking preformed PF4/UFH complexes with APAC

EP 4 149 488 B1

Figure 4

**Competing effect of APAC on the formation of ULICs of KKO/PF4/UFH**

Figure 5

**Dissociating effect of APAC on the preformed ULICs of KKO/PF4/UFH**

Figure 6

TF production by THP-1 cells incubated with PF4/KKO ± APAC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2015069327 W

- WO 2016030316 A

**Non-patent literature cited in the description**

- **AREPALLY et al.** *Blood.*, 2000, vol. 95, 1533-1540, https://www.ncbi.nlm.nih.gov/pubmed/10688805